# EUROPEAN PATENT SPECIFICATION

(11) **EP 2 895 047 B1**
(45) Date of publication and mention of the grant of the patent: **24.10.2018**
(21) Application number: 13836474.0
(22) Date of filing: 13.09.2013
(51) Int. Cl.: A61B 1/005, A61M 25/10, A61B 1/307

(54) **4-WAY CYSTOSCOPY CATHETER WITH LOW PROFILE BALLOON**
4-WEGE-ZYSTOSKOPIEKATHETER MIT FLACHEM BALLON
CATHÉTER À 4 VOIES POUR CYSTOSCOPIE DOTÉ D'UN BALLON À ENCOMBREMENT RÉDUIT

(30) Priority: 13.09.2012 US 201261700841 P
(43) Date of publication of application: 22.07.2015
(73) Proprietor: EMMY Medical, LLC, Holliston, MA 01746 (US)
(72) Inventor: GREENBERG, James, A., Weston, MA 02493 (US); ADAMS, Ronald, D., Holliston, MA 01746 (US); KOHLI, Neeraj, Natick, MA 01760 (US); HARARI, David, San Diego, CA 92103 (US)
(74) Representative: Arth, Hans-Lothar
(86) International application number: PCT/US2013/059822
(87) International publication number: WO 2014/043586

(56) References cited:
- WO-A2-2004/107951
- JP-A- H01 135 721
- US-A1- 2006 004 323
- US-A1- 2006 252 993
- US-A1- 2008 312 490
- US-A1- 2009 005 741
- US-A1- 2009 062 872
- US-A1- 2009 318 757
- US-B2- 6 689 148
- US-B2- 7 063 679
- 'Foley balloon catheter surucath' 2006, XP008179825 Retrieved from the Internet: <URL:www.suru.com/fole.htm> [retrieved on 2013-11-26]

## Description

### BACKGROUND

### Field of the Invention

This invention relates generally to a catheter for draining the urinary bladder configured to allow for transcatheter cystoscopy.

A urinary bladder tube is used in certain patients who have undergone major surgery, or any patient who is unable to urinate. There are many causes for inability to urinate. These causes differ with age and sex. For example, a small child may not urinate because of some congenital abnormality obstructing the bladder neck or urethra. In females, inability to urinate occurs in neurological diseases, after delivery of a baby, or after major abdominal or pelvic surgery. In a male, the most common causes of inability to urinate are an obstructing prostate, neurological diseases, or after major abdominal or pelvic surgery.

It can be desirable to continuously drain the bladder after major surgery at least for the purpose of monitoring the hourly urine output. It is also desirable to continuously drain the bladder by an indwelling Foley catheter in medical conditions where the measurement of hourly urine output is important to the well-being of the patient.

It can also be desirable to drain the bladder by an indwelling catheter after prostate or bladder surgery. Diverting the urine and blood will promote fast healing and prevent clots from building up in the bladder, which often cause more bleeding and severe pain.

It can also be important to drain the bladder by an indwelling catheter for pelvic surgeries. By draining the bladder, the operating space in the pelvis can be increased and it can facilitate access to structures such as the uterus in female patients.

It is also recognized that it can be recommended in clinical practice after pelvic surgeries to conduct an assessment of the bladder and ureters to ensure that no damage to these organs has occurred. In many cases an optimal way for making this assessment is the use of a cystoscope where the internal surface of the bladder can be viewed as well as the vesicoureteral junction. The vesicoureteral junction can be observed to look for flow of urine into the bladder indicating that no impairment of the ureters, bladder, or other structures has occurred during the surgery. In some cases, use of an intravenous dye such as indigo carmine can be used to visualize urinary tract flow and determine possible leakage. In the U.S. today, there are over 1 million gynecologic pelvic procedures performed each year. The incidence of urologic injury has been reported to range from 1 to 3%. During laparoscopic hysterectomy, the incidence of ureteral injury has been reported at 0.2 to 4.3% . Reoperation, when necessary if an undetected injury has occurred, carries an average expense of $8,000-$12,000. Today, many surgeons defer the urologic assessment due to costs, risks, inconvenience and the additional time required.

Currently, cystoscopy is performed through a 3 piece rigid metal system which needs assembly and availability of reusable sterilized equipment in the operating room. The procedure has remained largely unchanged for over 3 decades. The system is cumbersome, complicated, expensive, and not always available. In addition, it requires increased time of catheter removal and cystoscope assembly as well as multiple instances of urethral instrumentation, increasing risk of infection and urethral trauma. Systems and methods as disclosed herein can obviate these shortcomings.

In certain bladder or prostate surgeries, continuous bladder irrigation is used. This is achieved by injecting fluid continuously into the bladder and simultaneously draining the bladder. This type of Foley catheter is called a three-way catheter. One port will serve as fluid injection port into the bladder. The second port is for continuous drainage of the bladder content into a large urine bag. The third port is for a valve mechanism where a balloon is inflated inside the bladder to keep the tip of the Foley catheter indwelling inside the bladder.

In certain patients, the bladder must be drained for many years, such as debilitated patients or those with neurological or spinal cord lesions. If the bladder is not drained, the pressure in the bladder will build up and the kidneys will be obstructed. Continuous bilateral kidney obstruction may lead to renal failure.

Therefore, the use of an indwelling catheter can be clinically advantageous, and could be life-saving, both in an acute and chronic long term settings. Some examples of Foley catheters are disclosed, for example, in U.S. Pat. Nos. 5,810,790 to Ebling et al. and 6,096,013 to Hakky et al..

Document JP-H-01 135 721 discloses a catheter according to the preamble of claim 1.

### SUMMARY OF THE INVENTION

Disclosed herein is a 4-Way indwelling catheter according to claim 1. The catheter can function as a traditional Foley catheter at the start of a procedure in which the bladder is drained to provide optimum access and visualization of the pelvis. At the point in the procedure when the post-procedure cystoscopy is to be performed, the 4-Way Foley catheter eliminates the need to remove the original Foley catheter. The 4-Way Foley can include a balloon and inflation channel to stabilize the catheter in the bladder; a central lumen with a one way valve to enable the insertion of a cystoscope; an inflow port with a control valve; and an outflow port to enable bladder drainage. In some embodiments, the elongate body of the catheter distally beyond the proximal ports is limited to no more than, or exactly two lumens - the balloon inflation lumen and the central fluid/drainage/instrument lumen.

A method of performing a urologic or gynecologic procedure such as cystoscopy is also described, comprising cannulating the urethra with a catheter, the catheter comprising: a proximal end, a distal end having at least one exit port, and a flexible elongate tubular body therebetween; an inflatable balloon near the distal end of the catheter, wherein the balloon in its expanded configuration has a radial dimension that is at least about 1.5x of its axial dimension; advancing the catheter distally into the bladder; flowing media into a balloon proximate the distal end of the catheter such that the balloon when expanded has a radial dimension that is at least about 1.5x of its axial dimension; and advancing an instrument, such as a cystoscope, through a proximal port of the catheter, through the flexible elongate tubular body, and through the exit port of the catheter.

### BRIEF DESCRIPTION OF THE DRAWINGS

FIG. 1 is a side elevation view of a balloon-retained catheter, according to some embodiments of the invention.
FIG. 2 is a side elevation view of a balloon-retained catheter having a lumen for accommodating an endoscope, according to some embodiments of the invention.
FIG. 3 is an enlarged cross sectional view of the distal portion of the catheter as shown in FIG. 2, according to some embodiments of the invention.
FIG. 4 is a cross sectional view taken along line 4-4 of FIG. 3.
FIG. 5 illustrates a four-way cystoscopy catheter, according to some embodiments of the invention.
FIG. 6 illustrates another embodiment of a four-way cystoscopy catheter.
FIGS. 7A-7E illustrate various embodiments of distal tip catheter configurations.
FIG. 8 illustrates another embodiment of a 4-way cystoscopy catheter.
FIGS. 9A-9D illustrates an embodiment of a keyed catheter scope port and its attachment to an endoscope, such as a cystoscope.
FIGS. 10A-10B illustrate cross-sections through a balloon catheter, according to some embodiments of the invention.
FIGS. 11A-11D and 12 illustrate an indwelling catheter that can be steerable and curvable via a deflectable distal end, according to some embodiments of the invention.
FIG. 13 illustrates schematically an indwelling catheter passing through the urethra and into the bladder with a pancake-shaped balloon in an expanded configuration, and a cystoscope extending distally out of the distal end of the catheter.

### DETAILED DESCRIPTION

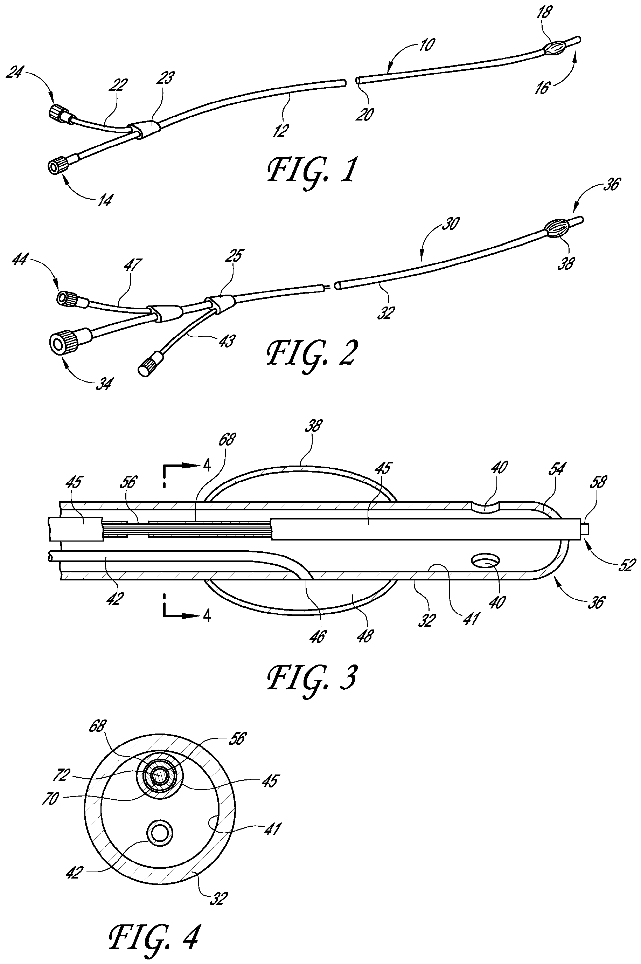

Referring to FIG. 1, a balloon-retained catheter 10, here being a Foley catheter, is shown. The catheter 10 includes a length of tubing 12 having a first proximal port 14 and an open distal end 16, with the distal opening including laterally and/or distally disposed apertures. Immediately behind the distal end 16 is an expandable member, e.g., an inflatable balloon 18 surrounding a portion of the tubing 12. A central lumen 20 defined by the tubing 12 extends from at least the hub 23 proximally to the distal end 16 thereof for conveying fluid therethrough. A second lumen 22 is disposed within the tubing 12 and is accessible via a second proximal port 24. The second lumen 22 leads to the interior of the balloon 18 for passage of fluid for balloon inflation and deflation. In some embodiments (not shown), a catheter may be in the form of a 3-way catheter and can include a third proximal port which can be used for continuous bladder irrigation, for example, in communication with a central lumen 20 distally past the proximal hub 23.

Referring now to FIGS. 2 through 4, a balloon-retained catheter 30 is shown wherein an instrument, e.g., an endoscope can be retained. The catheter 30 can be identical or similar to the catheter 10 of FIG. 1, except with the capability of housing an endoscope as hereafter described. The catheter 30 includes a length of tubing 32 having a proximal end 34 and an open distal end 36, with the distal end opening being four uniformly laterally disposed apertures as exemplified by aperture 40. Immediately behind the distal end 36 is an inflatable balloon 38 surrounding a portion of the tubing 32. A first lumen 41 defined by the tubing 32 extends from the proximal end 34 to the distal end 36 for conveying fluid therethrough. A second lumen 42 is disposed within the tubing 32 and is accessible via an open proximal end 44 situated in connector tubing 47. The second lumen 42 has an open distal end 46 leading to the interior 48 of the balloon 38 for passage of fluid to inflate or deflate the balloon 38.

A third lumen 45 is disposed within the tubing 32 and is accessible via an open proximal end 50 situated distally from the proximal end of the tubing 32 in lateral connector tubing 43. The third lumen 45 has an open distal end 52 at the distal tip 54 of the tubing 32, and creates a working channel that can accommodate an endoscope 56 or a similarly sized surgical instrument. In certain embodiments, the distal end 58 of the endoscope 56 projects beyond the distal tip 54 a distance sufficient to provide beneficial viewing, tissue engagement, and/or proximity to create a desired diagnostic or therapeutic result. In some embodiments, the first lumen 41 and third lumen 45 do not remain separate throughout their length but rather merge into a common central lumen distally past hub 25. As such, the elongate tubular body distal to the proximal ports, in some embodiments, has exactly 2 lumens, or no more than 2 lumens (a balloon inflation lumen and a fluid/urine/instrument lumen). A configuration allowing for a reduced number of lumens can means less boundary walls may be necessary, advantageously reducing the outside diameter of the catheter that is being passed through the patients' urethra. One benefit of the smaller outside diameter is that it can reduce pain and discomfort for the patient relating to the catheter.

Operation of the catheter 30 is commenced by first positioning the distal end 36 at a desired site usually within a cavity such as a urinary bladder. When desired placement is attained, fluid such as a saline solution is introduced into the second lumen 42 for travel to the interior 48 of the balloon 38 and inflation thereof and retention of the catheter 30 in place. Once so situated, the catheter 30 permits free fluid flow from and/or to the site of its distal end 36. To visually inspect the site of the distal end 36 of the catheter 30, an endoscope 56 is inserted into the third lumen 45 and advanced until its distal end 58 resides beyond the distal tip 54 of the tubing 32. Such advancement results in engagement of the port connector 60 and endoscope connector 62 for releasable retention of the endoscope 56 for use in viewing the catheterized site. In this manner, a physician or other healthcare worker can endoscopically view a site without first removing a catheterized site. In this manner, a physician or other healthcare worker can endoscopically view a site without first removing a catheter treating that site.

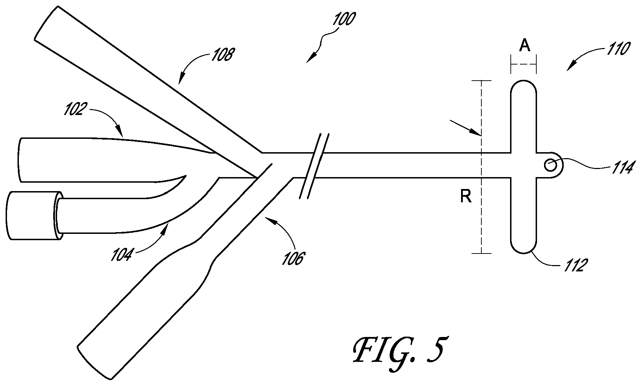

In some embodiments, disclosed herein is a 4-way cystoscopy catheter 100 designed to better allow cystoscopic evaluation of the bladder through an indwelling transurethral catheter, one embodiment of which is disclosed schematically in FIG. 5. In some embodiments, the device is a modification of the traditional 3-way urethral catheter, having four or more ports. However, low profile balloons as described herein can also be used in conjunction with conventional Foley and 3-way Foley catheters. The catheter body can be made of Silastic, latex, silicone, Teflon, or any other appropriate material. A first port can be a central port 102 having a one-way valve (not shown) that can be built in and designed to accommodate a cystoscope, such as a diagnostic cystoscope. The port 102 can advantageously serve as a protective channel to reduce the risk of friction and other trauma caused by placing an instrument directly into the urethra. In some embodiments, the instrument could be a catheter configured to emit energy, such as RF, microwave, laser, X-rays, ultrasound, thermal, and/or other types of energy. In some embodiments, the instrument could be a stiffening member, such as a wire, for use in, for example, an incontinence or prolapse treatment procedure in order to manipulate the anatomical location of the urethra while placing a mesh or another implant in another anatomical structure. In some embodiments, a first instrument is placed within the central lumen of the catheter and then removed, and then a second instrument is placed within the central lumen of the catheter. A second port can be a balloon inflation port 104; a third port can be a drainage port for urine 106; and a fourth port 108 can be a fluid inflow port. In some embodiments, ports 102, 106, 108 are fluidly connected to a single lumen, e.g., a central lumen in communication with one aperture s 114 on the distal end of the catheter, such that the catheter 100 has exactly 2 lumens (the central lumen and the balloon inflation lumen) distal to the proximal ports. In some embodiments, the central lumen could have a size of about or no more than about 22 French, 21 French, 20 French, 19 French, 18 French, 17 French, 16 French, 15 French, or 14 French. The aperture 114 is spaced distal-facing coaxial with the longitudinal axis of the catheter 100. The catheter 100 has a single, e.g., no more than one aperture (e.g., exit port). The single aperture is distally-facing and has the same or substantially the same diameter as that of the central lumen, which can advantageously eliminate the need for additional drainage apertures at the distal tip, potentially reducing catheter length required as well as manufacturing costs. In some embodiments, the central lumen can have a constant or substantially constant diameter throughout its entire length. The larger diameter single exit port can in some cases reduce the risk of obstruction from the particulates often seen within the bladder. In some embodiments, the exit port can include a mesh, filter, or other mechanism to further reduce the risk of obstruction from particulate matter. In some embodiments, the central lumen, outer diameter, and/or other portions of the catheter can include a therapeutic agent, such as an anesthetic agent, an anti-coagulant (e.g. heparin), an anti-inflammatory, an antimicrobial, and the like. Balloon inflation port 104 can be fluidly connected to a discrete balloon inflation lumen terminating at or near the distal end 110 of the catheter 100 within an expandable member such as a balloon 112, which can be a conventional spherical balloon or a low profile balloon in some embodiments. The balloon shape could be, for example, round, oval, flat, or cylindrical; symmetric or asymmetric along an axial, radial, or other axis. The low profile balloon 112 can be reversibly movable from an unexpanded to an expanded configuration by injection or removal of fluid from the balloon inflation port 104. Low profile balloon 112 can have an expanded radial dimension R that is at least about, about, or no more than about 1.25x, 1.5x, 1.75x, 2x, 2.5x, 3x, 4x, 5x, 6x, 7x, 8x, 9x, 10x, 12x, 15x, 20x, or more of the expanded axial dimension A (and/or height dimension) of the balloon 112, and sometimes have a "pancake"-like shape. In some embodiments, the expanded axial dimension A of the balloon 112 is about 10-20mm, such as about or less than about 20mm, 15mm, or 10mm.

In some embodiments, a balloon is a flat pancake-shape (i.e., the depth is less than the width; e.g., by a ratio of at least about, about, or no more than about 2:1, 3:1, 4:1, 5:1, 6:1, 7:1, 8:1, 9:1, 10:1, 20:1, or more). In some embodiments, a pancake-shaped balloon is wider than it is deep (e.g., at least about, about, or no more than about 1.5x wider than deep; 2× wider than deep; 5× wider than deep; 10×wider than deep; 25× wider than deep). In some embodiments, a balloon is tall and narrow (e.g., at least about, about, or no more than about 1.5× taller than wide; 2× taller than wide; 3× taller than wide; 5× taller than wide; 10× taller than wide; 25× taller than wide). The balloon can, in some embodiments, have an inflated volume of about or less than about 30cc, 10cc, 5cc, or 3cc. A catheter 100 having a low profile balloon can in some cases advantageously provide an improved and more comfortable seal proximate an anatomical site, e.g., the internal urethral orifice and thus prevent leakage around the catheter. In some cases, such a catheter can also advantageously be less view-obstructing/in the way of the cystoscope and as such provide improved visualization during a cystoscopy procedure, for both male and female anatomy.

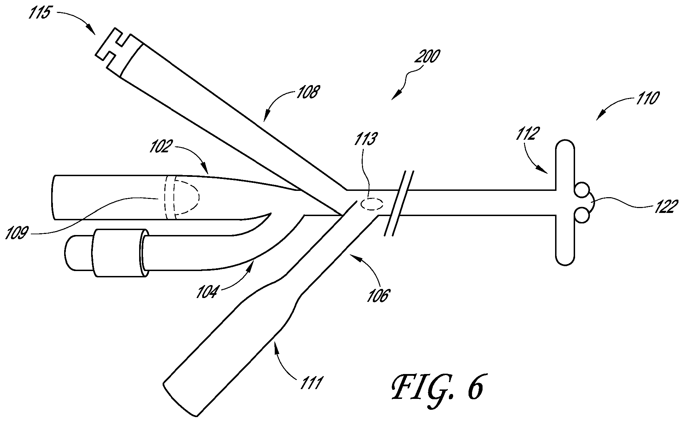

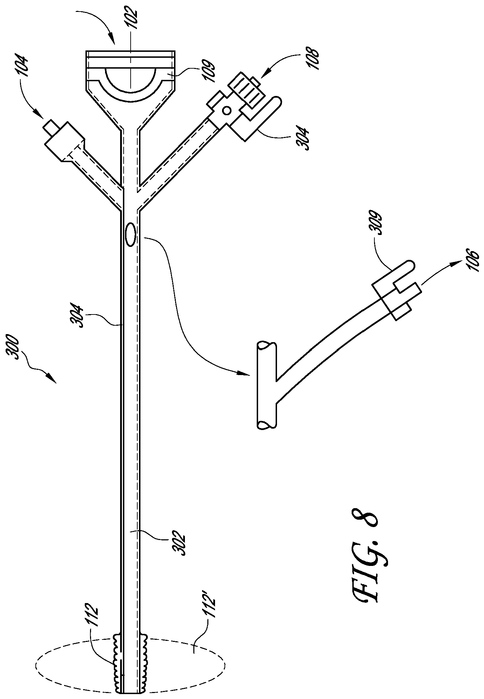

FIG. 6 illustrates a 4-way cystoscopy catheter 200 similar to that of FIG. 5, also illustrating a luer or other fitting 115 on the proximal end of the inflow port 108 for connection to an IV bag or another source of media. The instrument, e.g., cystoscope port 102 can include a valve 109 such as a one-way valve present, or other reversible sealing mechanism to prevent the leakage of fluids when the port is not occupied by the cystoscope. The outflow port 106 can include a clamp 111 such as a roller clamp to reversibly allow for urine/media drainage from the body lumen such as the bladder. As with the embodiment in FIG. 5, ports 102, 106, 108 can be fluidly connected to a single lumen in communication with a central lumen 113. The distal end 110 of the catheter can again have a balloon 112 such as a low-profile balloon, but the distal tip 122 could have rounded corners 117 to decrease the potential for trauma, or another configuration as shown close-up and described in connection with FIGS. 8A-E below.

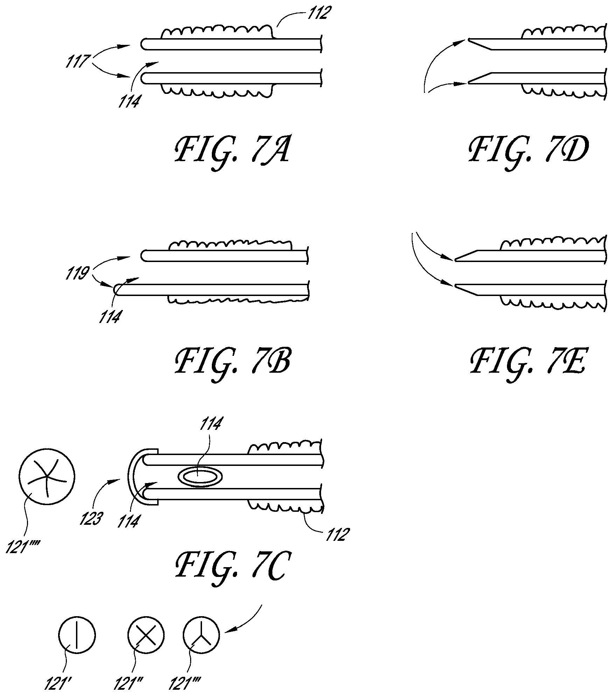

FIGS. 7A-E illustrate various embodiments of distal tip 122 catheter configurations with blunt tips to reduce or avoid trauma associated with the distal tip and/or cystoscope. Also illustrated is balloon 112 in an uninflated state on or near the sidewall of the distal end catheter, and an aperture 114 that is distally facing. As illustrated in FIG. 7A, include a distal tip 117 embodiment having rounded edges. FIG. 7B illustrates a distal tip embodiment 119 having a beveled edge. FIG. 7C illustrates a distal tip configuration having an end cap 123 surrounding the distally facing surface of the distal tip. The end cap 123 can include one or more slit patterns 121, such as a star pattern 121"", linear pattern 121', cross pattern 121", V or W pattern (not shown), Y pattern 121"', or others. The end cap 123 with slits 121 can advantageously provide a safety feature and also protect an endoscope while retracted within the catheter. Also shown are one, two, or more apertures 114 for media infusion and or drainage along the sidewall. FIG. 7D illustrates an embodiment of a contoured distal tip 125 having an internal bevel (with a decreasing axial length from a lateral to medial direction). FIG. 7E illustrates an embodiment of a contoured distal tip 127 having an external bevel (with an increasing axial length from a medial to lateral direction).

FIG. 8 illustrates another embodiment of a 4-way cystoscopy catheter 300, illustrating an endoscope port 102 at the proximal-most end and coaxial with the longitudinal axis of the catheter 300. Endoscope port 102 can include a valve 109 or other seal in the port lumen as previously described. Also illustrated are inflow ports 108 and outflow ports 106 which can include a luer lock and/or stopcock-type valve 309 as shown to regulate fluid inflow and outflow, respectively. In some embodiments, the ports can have indicia for enhanced identification, such as be differing colors. Ports 102, 106, 108 can also all be in fluid communication with a central lumen 302 as previously described. Also shown is balloon inflation port 104 which can be in communication with a discrete balloon inflation lumen 304 separate from the central lumen 302. Balloon inflation lumen 304 can be in turn be connected to a balloon 112 which can be a conventional or low profile, e.g., "pancake" type balloon as previously described, and shown in an uninflated 112 and inflated configuration 112' (in phantom).

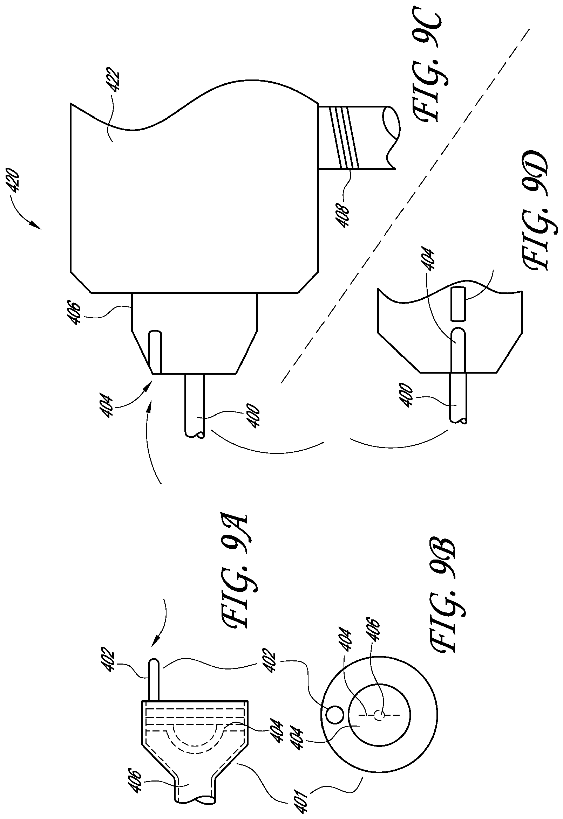

FIGS. 9A-9D illustrates an embodiment of a keyed catheter scope port 401 and its attachment to an endoscope 420, such as a cystoscope. FIG. 9A is a side view of scope port 401 including one, two, or more alignment pins 402 and internal seal or valve 404 across the scope port lumen 406. The alignment pin 402 could include indicia, such as a color to match with corresponding indicia on an endoscope pin slot 402. FIG. 9B illustrates an end view of the scope port 102 illustrated in FIG. 9A. FIG. 9C is a side view of the endoscope 420 showing scope proximal portion 422 which can be operably connected to a light post 408. Also illustrated is scope hub 406 including one, two, or more pin slots 404 complementary to and configured to reversibly mate with the alignment pins 420. Other locking mechanisms other than pins and slots are also within the scope of the invention. FIG. 9D is a top view of the embodiment of FIG. 9C. The keyed catheter system can be advantageous in some embodiments to help provide spatial orientation of certain anatomical landmarks while navigating the endoscope within the body. For example, using the orientation of a pin-slot combination as a guide relative to a clock face, an operator could identify the left ureter with respect to a right ureter. Furthermore, such embodiments can still advantageously allow for relative movement between the scope and the catheter.

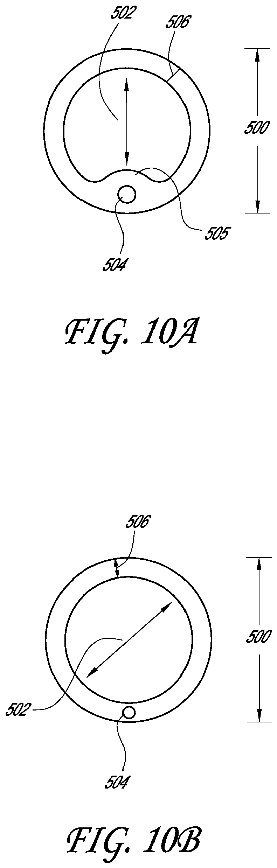

In some embodiments, a catheter could have an elongate shaft portion of which the outer diameter 500 of the shaft is between about 5mm and about 7mm, such as about 6mm. The inner diameter 502 of the central lumen could be, for example, about 2mm about 4mm, such as between about 2.3mm and about 2.6mm, or between about 3.0mm and about 3.5mm. The central lumen could, for example, have a circular cross-sectional area as illustrated in FIG. 10A, or could have a localized raised area 505 to provide more room for the balloon inflation lumen 504 as illustrated in FIG. 10B. The balloon inflation lumen 504 could, for example, have a diameter of between about 0.6mm and about 1.2mm, or between about 0.8mm and about 1.0mm for example. The shaft sidewall could have a thickness 506 of between about 1mm and about 2mm, such as between about 1.2mm and about 1.5mm, for example.

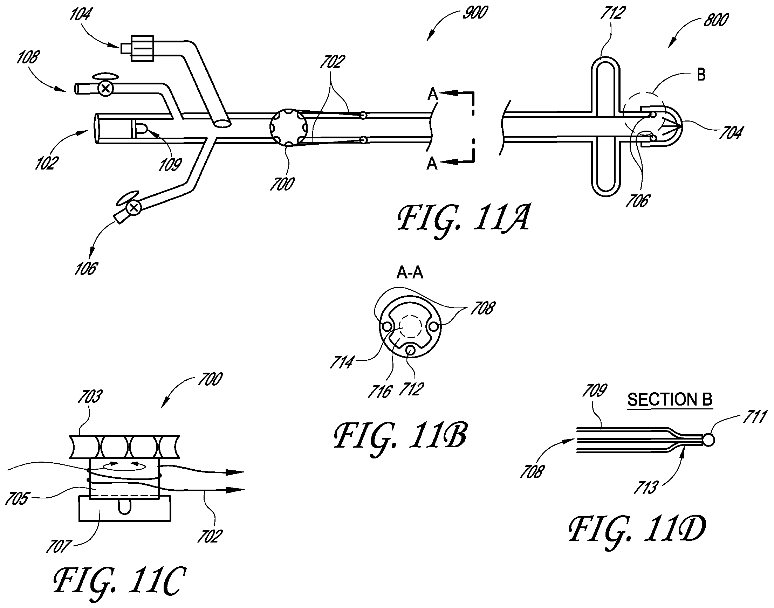

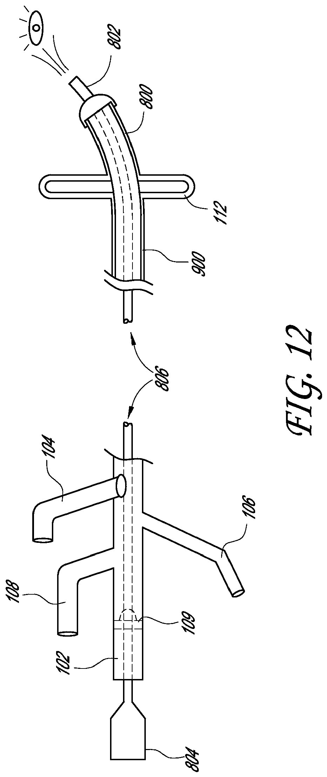

FIGS. 11A-11D illustrates an indwelling catheter that can be steerable and curvable via a deflectable distal end. Catheters as described herein could be configured to house a rigid rod lens scope (e.g., a 70 degree scope) or a flexible fiber scope (e.g., a 0 degree scope). FIG. 11A illustrates a steerable catheter 900 having any of balloon inflation port 104, inflow port 108, outflow port 106, and scope ports 102 with valve/seal 109 that can be as previously described. At the proximal end or along the shaft of the catheter can be a deflection control 700 for deflecting the distal end of the catheter through a controllable angular range. The deflection control 700 could be, for example, a rotatable knob operably connected to one, two, or more control wires, which travel within control wire lumens 708 and are operably attached at their distal end to the distal end of the catheter, such as at a distal cap 704. FIG. 11B is a cross-section through line A-A of FIG. 11A, illustrating central lumen 714 with scope 716 therethrough, balloon inflation lumen 712, and control wire lumens 708. FIG. 11C illustrates schematically deflection control 800 having a rotatable knob 703, base 707, and body 705 of which the proximal ends of control wires 702 can be wrapped around. Rotation of the knob 703 in an appropriate direction can move the distal end of the catheter from an undeflected configuration in which the distal end is coaxial with the longitudinal axis of the catheter to a deflected configuration in which the distal end is not coaxial with the longitudinal axis of the catheter. The distal end of the catheter can be configured to deflect up to about 45, 60, 90 degrees, or more. FIG. 11D illustrates a section through the distal end of the catheter, showing catheter sidewall 709 which can be collapsed distally near 713 onto the pullwires 708 as shown, e.g., using heat, and terminating in ball 711. Alternatively, the lumen can be filled with a material such as a resin to lock the wires 708 to the catheter wall 709. FIG. 12 shows the catheter 900 of FIGS. 11A-11D in a deflected configuration, showing a flexible fiber 0 degree scope 804, scope shaft 806, deflected catheter distal tip 800, and scope tip 802 extending beyond the deflected catheter tip 800.

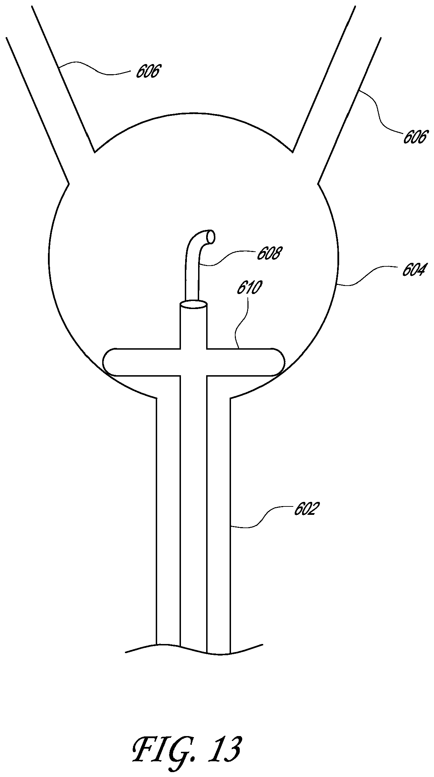

FIG. 13 illustrates schematically an indwelling catheter passing through the urethra 602 and into the bladder 604 with a pancake-shaped balloon 610 in an expanded configuration, and instrument such as a cystoscope 608 extending distally out of the distal end of the catheter. The distal end of the cystoscope can be rigid and coaxial with the longitudinal axis of the catheter, or deflected as shown. Also shown are ureters 606.

It is contemplated that various combinations or subcombinations of the specific features and aspects of the embodiments disclosed above may be made and still fall within one or more of the inventions. Further, the disclosure herein of any particular feature, aspect, method, property, characteristic, quality, attribute, element, or the like in connection with an embodiment can be used in all other embodiments set forth herein. Accordingly, it should be understood that various features and aspects of the disclosed embodiments can be combined with or substituted for one another in order to form varying modes of the disclosed inventions. Thus, it is intended that the scope of the present inventions herein disclosed should not be limited by the particular disclosed embodiments described above. Moreover, while the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents, and alternatives falling within the spirit and scope of the various embodiments described and the appended claims. Any methods disclosed herein need not be performed in the order recited. The methods disclosed herein include certain actions taken by a practitioner; however, they can also include any third-party instruction of those actions, either expressly or by implication. For example, actions such as "passing an instrument through a central lumen of a catheter" include "instructing the passing of an instrument through the central lumen of a catheter." The ranges disclosed herein also encompass any and all overlap, subranges, and combinations thereof. Language such as "up to," "at least," "greater than," "less than," "between," and the like includes the number recited. Numbers preceded by a term such as "approximately", "about", and "substantially" as used herein include the recited numbers, and also represent an amount close to the stated amount that still performs a desired function or achieves a desired result. For example, the terms "approximately", "about", and "substantially" may refer to an amount that is within less than 10% of, within less than 5% of, within less than 1% of, within less than 0.1% of, and within less than 0.01% of the stated amount.

## Claims

1. An indwelling cystoscopy catheter (100), comprising:
a proximal end, a distal end (110), and a flexible elongate tubular body therebetween;
an inflatable balloon (112) near the distal end of the catheter and proximal to a distal tip (122) of the flexible elongate tubular body,
wherein the distal tip (122) comprises a single exit port (114) for fluid inflow, fluid outflow, and passage therethrough of a cystoscope the single exit port being distally facing,
wherein the balloon (112) is transformable from an unexpanded and an expanded configuration;
a first proximal port (108) for fluid inflow;
a second proximal port (106) for fluid outflow;
a third proximal port (104) for balloon inflation, in fluid connection with the balloon (112); **characterized in that** the balloon is a low profile balloon and in its expanded configuration comprises an oblong shape having a radial dimension that is at least 1.5x of its axial dimension and the catheter further comprises a fourth proximal port (102) for insertion of a cystoscope therethrough, the fourth proximal port including a sealing member (109) therein to prevent proximal backflow of fluid,
wherein the first, second, and fourth proximal ports (108, 106, 102) are fluidly connected to a common central lumen (113) extending through the elongate tubular body to the single exit port (114) of the distal tip (122) and the single exit port has the same diameter as that of the central lumen.

2. The catheter (100) of Claim 1, wherein the balloon (112) in its expanded configuration has a radial dimension that is at least 2x of its axial dimension.

3. The catheter (100) of Claim 1 or Claim 2, wherein the balloon (112) in its expanded configuration has a volume of no more than 5cc.

4. The catheter (100) of any of the Claims 1 - 3, wherein the distal tip (117, 119) comprises a rounded shape or a beveled shape.

5. The catheter (100) of any of the Claims 1 - 4, wherein the distal end (110) comprises an end cap (123) having a slot (121) therein.

6. The catheter (100) of any of the Claims 1 - 5, wherein the distal end (110) is movable from a first configuration coaxial with a longitudinal axis of the elongate tubular body to a second configuration not coaxial with the longitudinal axis of the elongate tubular body.

7. The catheter (100) of Claim 6, wherein the second configuration comprises a curved state.

8. The catheter (100) of any of the Claims 6 or 7, further comprising a deflection control (700) on the elongate tubular body.

9. The catheter (100) of Claim 8, wherein the deflection control (700) comprises a rotatable knob (703).

10. The catheter (100) of Claim 8 or 9, further comprising at least one pullwire (708) operably connected proximally to the deflection control (700) and operably connected distally to the distal end (110) of the catheter.

## Patentansprüche

1. Ein Zystoskopie-Dauerkatheter (100), umfassend:
ein proximales Ende, ein distales Ende (110) und dazwischen einen flexiblen länglichen Rohrkörper;
einen aufblasbaren Ballon (112) nahe dem distalen Ende des Katheters und proximal zu einer distalen Spitze (122) des flexiblen länglichen Rohrkörpers,
wobei die distale Spitze (122) eine einzige Austrittsöffnung (114) zum Fluidzufluss, Fluidabfluss, und zur Durchführung eines Zystoskops, wobei die einzige Austrittsöffnung distal zugewandt ist,
wobei der Ballon (112) aus einer nicht expandierten und einer expandierten Konfiguration umwandelbar ist;
einen ersten proximalen Anschluss (108) für den Fluidzufluss;
einen zweiten proximalen Anschluss (106) für den Fluidabfluss;
einen dritten proximalen Anschluss (104) zur Balloninflation, der in Fluidverbindung mit dem Ballon (112) steht; **dadurch gekennzeichnet, dass** der Ballon ein Ballon mit niedrigem Querschnitt ist und in seiner expandierten Konfiguration eine längliche Form mit einer radialen Abmessung umfasst, die mindestens 1,5-fache seiner axialen Abmessung beträgt, und der Katheter ferner umfasst
einen vierten proximalen Anschluss (102) zur Einführung eines Zystoskops durch diesen Anschluss, wobei der vierte proximale Anschluss darin ein Dichtungselement (109) zur Verhinderung eines proximalen Fluidrückflusses enthält,
wobei der erste, zweite und vierte proximale Anschluss (108, 106, 102) in Fluidverbindung mit einem gemeinsamen zentralen Lumen stehen (113), der sich durch den länglichen Rohrkörper zu der einzigen Austrittsöffnung (114) der distalen Spitze (122) erstreckt und die einzige Austrittsöffnung den gleichen Durchmesser wie das zentrale Lumen aufweist.

2. Katheter (100) gemäß Anspruch 1, wobei der Ballon (112) in seiner expandierten Konfiguration eine radiale Abmessung aufweist, die mindestens das 2-fache seiner axialen Abmessung beträgt.

3. Katheter (100) gemäß Anspruch 1 oder 2, wobei der Ballon (112) in seiner expandierten Konfiguration ein Volumen von nicht mehr als 5 cm³ aufweist.

4. Katheter (100) gemäß einem der Ansprüche 1 - 3, wobei die distale Spitze (117, 119) eine abgerundete Form oder eine abgeschrägte Form umfasst.

5. Katheter (100) gemäß einem der Ansprüche 1 - 4, wobei das distale Ende (110) eine Endkappe (123) mit einem Schlitz (121) darin umfasst.

6. Katheter (100) gemäß einem der Ansprüche 1 - 5, wobei das distale Ende (110) von einer ersten Konfiguration, die koaxial mit einer Längsachse des länglichen Rohrkörpers ist, in eine zweite Konfiguration, die nicht koaxial mit der Längsachse des länglichen Rohrkörpers ist, beweglich ist.

7. Katheter (100) gemäß Anspruch 6, wobei die zweite Konfiguration einen gekrümmten Zustand umfasst.

8. Katheter (100) gemäß einem der Ansprüche 6 oder 7, ferner umfassend eine Ablenksteuerung (700) an dem länglichen Rohrkörper.

9. Katheter (100) gemäß Anspruch 8, wobei die Ablenksteuerung (700) einen drehbaren Knopf (703) umfasst.

10. Katheter (100) gemäß Anspruch 8 oder 9, ferner umfassend mindestens einen Zugdraht (708), der proximal mit der Ablenksteuerung (700) betriebsmäßig verbunden ist und distal mit dem distalen Ende (110) des Katheters betriebsmäßig verbunden ist.

## Revendications

1. Une sonde à demeure de cystoscopie (100), comprenant les éléments suivants :
une extrémité proximale, une extrémité distale (110) et un corps tubulaire flexible et extensible entre ces deux extrémités ;
un ballonnet gonflable (112) à proximité de l'extrémité distale du cathéter et situé de façon proximale par rapport à l'embout distal (122) du corps tubulaire flexible et extensible,
l'embout distal (122) comprenant un orifice de sortie unique (114) destiné à l'entrée et à la sortie du liquide, ainsi qu'au passage par un cystoscope ; l'orifice de sortie unique faisant face au plan distal,
où le ballonnet (112) est une forme transformable d'une configuration non expansée à une configuration expansée ;
un premier orifice proximal (108) pour l'entrée du liquide ;
un deuxième orifice proximal (106) pour la sortie du liquide ;
un troisième orifice proximal (104) pour le gonflage du ballonnet, le liquide étant connecté au ballonnet (112); **caractérisé par le fait que** le ballonnet soit un ballonnet à profil discret et qu'il présente une forme oblongue dans sa configuration expansée présentant une dimension radiale d'au moins 1,5 x sa dimension axiale ; le cathéter comprenant également
un quatrième orifice proximal (102) pour l'insertion d'un cystoscope, le quatrième orifice proximal comprenant un élément d'étanchéité (109) afin d'empêcher le reflux proximal du liquide ;
où le premier, le deuxième et le quatrième orifices proximaux (108, 106, 102) sont connectés du point de vue du liquide à une lumière centrale commune (113) s'étendant le long du corps tubulaire extensible jusqu'à l'orifice de sortie unique (114) de l'embout distal (122), l'orifice de sortie unique présentant le même diamètre que la lumière centrale.

2. Le cathéter (100) selon la revendication 1, où le ballonnet (112) se trouve dans sa configuration expansée et présente une dimension radiale d'au moins 2 x sa dimension axiale.

3. Le cathéter (100) selon la revendication 1 ou 2, où le ballonnet (112) se trouve dans sa configuration expansée et présente un volume inférieur à 5 cm³.

4. Le cathéter (100) selon l'une des revendications 1 à 3, où l'embout distal (117, 119) présente une forme arrondie ou biseautée.

5. Le cathéter (100) selon l'une des revendications 1 à 4, où l'extrémité distale (110) comprend un capuchon d'extrémité (123) doté d'une fente (121).

6. Le cathéter (100) selon l'une des revendications 1 à 5, où l'extrémité distale (110) peut être déplacée d'une première configuration coaxiale avec un axe longitudinal du corps tubulaire extensible à une seconde configuration non coaxiale avec l'axe longitudinal du corps tubulaire extensible.

7. Le cathéter (100) selon la revendication 6, où la seconde configuration comprend une courbure.

8. Le cathéter (100) selon l'une des revendications 6 ou 7, comprenant également une commande de déflexion (700) sur le corps tubulaire extensible.

9. Le cathéter (100) selon la revendication 8, où la commande de déflexion (700) comprend un bouton rotatif (703).

10. Le cathéter (100) selon la revendication 8 ou 9, comprenant également au moins un fil à tirer (708) connecté de manière opérationnelle et proximale par rapport à la commande de déflexion (700) et connecté de manière opérationnelle et distale par rapport à l'extrémité distale (110) du cathéter.
